(19) Europäisches Patentamt
European Patent Office
Office européen des brevets

(11) Numéro de publication: **0 001 021**
**B1**

(12) # FASCICULE DE BREVET EUROPEEN

(45) Date de publication du fascicule de brevet: **16.09.81**

(21) Numéro de dépôt: **78400046.5**

(22) Date de dépôt: **30.06.78**

(51) Int. Cl.³: **C 07 D 471/22,**
A 61 K 31/475
//(C07D471/22, 221/00,
221/18, 221/00, 209/00)

(54) **Nouveaux dérivés d'alcaloides pentacycliques, procédé de préparation, application à la synthèse de produits du groupe de l'éburnamonine et compositions pharmaceutiques.**

(30) Priorité: **25.07.77 FR 7722747**

(43) Date de publication de la demande:
**07.03.79 Bulletin 79/5**

(45) Mention de la délivrance du brevet:
**16.09.81 Bulletin 81/37**

(84) Etats Contractants Désignés:
**BE CH DE GB NL**

(56) Documents cités:
**NL - A - 75 10315**

**BULLETIN DE LA SOCIETE CHIMIQUE DE FRANCE, 1976, NO. 11—12, 1961—62**

(73) Titulaire: **ROUSSEL-UCLAF**
**102, route de Noisy Boîte postale no.9**
**F-93230 Romainville (FR)**

(72) Inventeur: **Warnant, Julien**
**13, rue Jacques Dulud**
**F-92200 Neuilly sur Seine (FR)**
Inventeur: **Farcilli, André**
**1, Allée Vincent d'Indy**
**F-93110 Rosny sous Bois (FR)**
Inventeur: **Medici, Italo**
**7, rue Bernard de Jussieu**
**F-93140 Bondy (FR)**

(74) Mandataire: **Fritel, Hubert et al,**
**102, route de Noisy Boîte Postale No 9**
**F-93230 Romainville (FR)**

**EP 0 001 021 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

Nouveaux dérivés d'alcaloïdes pentacycliques, procédé de préparation, application à la synthèse de produits du groupe de l'éburnamonine et compositions pharmaceutiques.

La présente invention concerne de nouveaux dérivés d'alcaloïdes pentacycliques, leur procédé de préparation, leur application à la synthèse de produits du groupe de l'éburnamonine et les compositions pharmaceutiques les renfermant.

Dans le Bulletin de la Société Chimique de France, 1976, n° 11—12, p. 1961—2, on décrit la préparation de dérives du groupe de l'éburnamonine selon le schéma suivant:

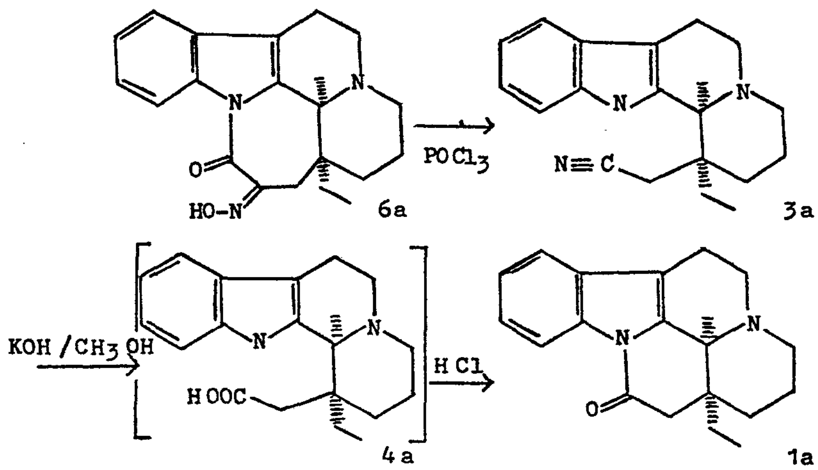


6a 3a 4a 1a

$POCl_3$ provoque un réarrangement de Beckmann; on obtient un nitrile que est traité ensuite successivement par $KOH/CH_3$ OH puis HCl pour donner la vincamone 1a ($[\alpha]_D = -100°$ ($CHCl_3$,C = 0,783).

Cette suite de réactions peut être réalisée sans isolement du nitrile 3a (rendement: 44%).

La demande de brevet hollandais NL—A—75 103 15 (Sandoz A—G) décrit des produits de formule:

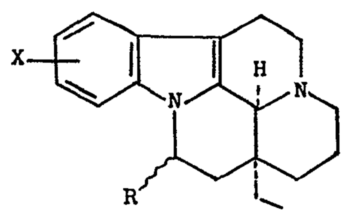

dans laquelle, notamment, X peut représenter un atome d'hydrogène, et R un radical amino, et leur application en thérapeutique, notamment pour lutter contre les dommages vasculaires cérébraux.

La présente invention a pour objet les produits de formule I

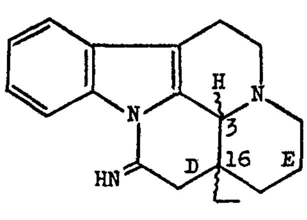

lesdits produits de formule I étant sous leurs formes racémiques ou optiquement actives, ainsi que les sels d'addition avec les acides minéraux ou organiques desdits produits de formule I.

Dans les produits de formule I, l'atome d'hydrogène en position 3 et le radical éthyle en position 16, peuvent chacun occuper l'une ou l'autre des orientations $\alpha$ et $\beta$, ce qui détermine l'existence de diastéréoisomères cis et trans.

Ceci revient à dire que dans les produits de formule I, la jonction des cycles D/E peut être cis ou trans.

Les sels d'addition avec les acides minéraux ou organiques des produits de formule I, peuvent être, par exemple, les sels formés avec les acides chlorhydrique, bromhydrique, iodhydrique, nitrique, sulfurique, phosphorique, acétique, formique, benzoïque, maléique, fumarique, succinique, tartrique, citrique, oxalique, glyoxylique, aspartique, ascorbique, les acides alcoylmonosulfoniques, tels que l'acide méthanesulfonique, l'acide éthanesulfonique, l'acide propanesulfonique, les acides alcoyldisulfoniques tels que l'acide méthanedisulfonique, l'acide $\alpha,\beta$-éthanedisulfonique, les acides arylmonosulfoniques, tel que l'acide benzènesulfonique et les acides aryldisulfoniques.

Parmi les produits, objet de l'invention, on retient plus particulièrement les produits tels que définis par la formule I ci-dessus, caractérisés en ce que dans la formule I, l'atome d'hydrogène en position 3 et le radical éthyle en position 16 sont cis, ainsi que leurs sels d'addition avec les-acides minéraux ou organiques.

Dans lesdits produits de formule I préférés, l'atome d'hydrogène en position 3 et le radical éthyle en position 16 sont cic l'un par rapport à l'autre, ce qui revient à dire que, dans ces produits, la jonction des cycles D et E est cis.

L'invention a tout spécialement pour objet le (3$\alpha$, 16$\alpha$) 14-imino-(15 H) éburnaménine.

Selon l'invention, les produits tels que définis par la formule I ci-dessus, sous leurs formes racémiques ou optiquement actives, ainsi que les sels d'addition avec les acides minéraux ou organiques desdits produits de formule I, peuvent être préparés par un procédé caractérisé en ce que l'on traite un produit de formule II

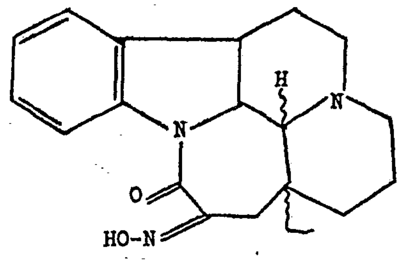

(II)

ou un de ses sels, par un agent basique pour obtenir un produit de formule I correspondant que, si désiré, on traite par un acide minéral ou organique pour en former le sel.

La transformation des produits de formule II en produits de formule I n'affecte pas la conformation de la molécule. C'est ainsi que si l'on part d'un produit de formule II de configuration *cis* (jonction des cycles D et E) on obtient produit I de configuration *cis*.

Le produit de formule II utilisé peut se présenter sous forme racémique ou optiquement active. Le produit de formule I obtenu à partir dudit produit de formule II se présente bien entendu sous la forme stéréochimique correspondante.

Selon l'invention, en particulier, le (3$\alpha$, 16$\alpha$) 14-imino-(15H)-éburnaménine et ses sels, peuvent être preparés par un procédé tel que décrit ci-dessus, caractérisé en ce que l'on utilise comme produit de formule II, la (3$\alpha$, 16$\alpha$) 14,15 dihydro 15-hydroxyimino D- homo éburnaménin -14-one ou l'un de ses sels.

Dans des conditions préférentielles de mise en oeuvre de l'invention, le procédé ci-dessus décrit est réalisé de la manière suivante:

— Les sels des produits de formule II utilisés éventuellement au départ du procédé peuvent être par exemple I chlorhydrate, le phosphate, le sulfate.

— Lorsque l'on utilise un sel de produit de formule II, on emploie un excès d'agent basique: l'agent basique doit être présent en quantité suffisante pour neutraliser le milieu et agir sur le produit de formule II.

L'agent basique utilisé peut être un hydroxyde de métal alcalin tel que l'hydroxyde de sodium, l'hydroxyde de potassium ou l'hydroxyde de lithium, ou encore l'hydroxyde de baryum.

La réaction du produit de formule II avec l'agent basique est réalisée à chaud et de préférence à une température allant de 100 à 150°C.

La réaction du produit de formule II avec l'agent basique peut être réalisée au sein d'un solvant organique tel qu'un alcool, et de préférence un alcool dont le point d'ébullition est supérieur à 100°C comme l'éthoxy éthanol, l'éthylène glycol, le diéthylène glycol, le triéthylène glycol, ou ou sein d'un solvant aprotique comme le diméthylsulfoxyde, le solvant organique choisi pouvant être utilisé en mélange avec l'eau. On opère de préférence à température de reflux du milieu réactionnel.

Le réaction du produit de formule II avec l'agent basique peut également être réalisée dans l'eau, de préférence à une température allant de 100 à 150°C; on opère alors sous pression.

Lorsque l'on prépare un produit de formule I en milieu aqueux, on utilise de préférence un faible volume d'eau, faute de quoi il se produit une hydrolyse simultanée, plus ou moins importante selon le volume d'eau utilisé, du produit de formule I, ce qui diminue le rendement de la réaction en produit de formule I.

L'hydrolyse simultanée de produit de formule I peut au contraire être mise â profit lorsque l'on cherche à préparer le produit d'hydrolyse du produit de formule I, produit de formule III

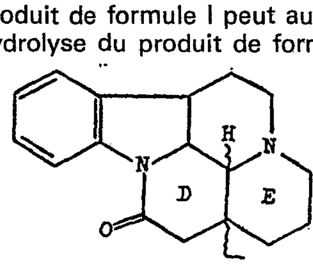

(III)

comme on le verra plus loin.

Les formes optiquement actives des produits de formule I tels que définis ci-dessus peuvent, en outre, être préparées par dédoublement des racémiques, selon les méthodes usuelles en la matière.

L'invention a également pour objet une application des produits de formule I telle que définie ci-dessus, et de leurs sels d'addition avec les acides minéraux et organiques, à la préparation des produits du groupe de l'éburnamonine, de formule III

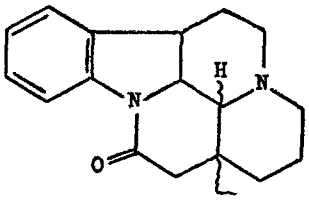

(III)

sous leurs formes racémiques ou optiquement actives, caractérisée en ce que l'on soumet un produit de formule I, ou un de ses sels, à l'action d'un agent d'hydrolyse, pour obtenir un produit de formule III sous forme salifiée ou non, et que, dans le cas où l'on obtient un sel de produit de formule III, l'on traite, le cas échéant, par une base pour obtenir le produit de formule III correspondant.

La transformation des produits de formule I en produits de formule III n'affecte pas la conformation de la molécule. C'est ainsi qui si l'on part d'un produit de formule I de configuration *cis* (jonction des cycles D et E) on obtient un produit III de configuration *cis*.

De même, si l'on part d'un produit I optiquement actif, on obtient un produit de formule III optiquement actif.

L'invention a, en particulier, pour objet, une application telle que définie ci-dessus, caractérisée en ce que le produit de formule I de départ est préparé selon le procédé décrit plus haut, et tout spécialement, une application, telle que définie ci-dessus, à la préparation de la ($3\alpha$, $16\alpha$) éburnaménin-14(15H)-one, caractérisée en ce que l'on utilise comme produit de formule I, le ($3\alpha$, $16\alpha$) 14-imino-(15H) éburnaménine.

Dans l'application de l'invention, l'hydrolyse du produit de formule I est réalisée en milieu acide ou en milieu basique.,

Elle est réalisée plus aisément en milieu acide.

L'acide utilisé peut être, par exemple, l'acide chlorydrique, l'acide sulfurique, l'acid phosphorique, l'acide formique.

L'hydrolyse en milieu acide peut être réalisée à température ambiante.

L'hydrolyse en milieu acide peut être réalisée dans l'eau, ou en présence d'un solvant organique tel qu'un alcool comme le méthanol, l'éthanol; ou au sein d'un mélange de ces solvants.

On peut réaliser également l'hydrolyse en milieu basique.

L'agent basique peut être, par exemple, un hydroxyde de métal alcalin, tel que l'hydroxyde de sodium, l'hydroxyde de potassium ou l'hydroxyde de lithium. Ce peut être également, par exemple, l'ammoniaque ou l'hydroxyde de baryum.

Lorsque l'on opère en milieu basique, l'hydrolyse est réalisée alors de préférence à une température allant de 100 à 150°C. On opère sous pression.

L'invention a, plus particulièrement, pour objet la préparation de produits de formule III telle que définie ci-dessus, qui consiste à préparer un produit de formule I, et qui est caractérisée en ce que, sans être isolé, le produit de formule I est transformé en produit de formule III.

On prépare alors un produit de formule I notamment à partir d'un produit II par action d'un agent basique, puis soumet le produit de formule I obtenu par exemple à une hydrolyse acide.

L'invention a, aussi plus particulièrement, pour objet la préparation de produits de formule III, telle que définie ci-dessus caractérisée en ce que, le produit de formula I est préparé en milieu aqueux selon le procédé décrit plus haut, et que l'hydrolyse dudit produit de formule I est réalisée au fur et à mesure de sa préparation.

La préparation du produit de formule I et son hydrolyse sont réalisées dans les conditions décrites plus haut pour la préparation de produits de formule I en milieu aqueux et l'hydrolyse des produits de formule I en milieu basique.

L'ensemble des produits de formule I, tels que définis ci-dessus ainsi que leurs sels d'addition avec les acides minéraux ou organiques, présentent d'intéressantes propriétés pharmacologiques: ce sont notamment des oxygénateurs et vasorégulateurs cérébraux de grande valeur; ils entraînent, en particulier, une augmentation du flux cérébral au niveau de la microcirculation cérébrale.

Ces propriétés justifient l'application en thérapeutique, à titre de médicaments, des produite tels que définis par la formule I ci-dessus, sous leurs formes racémiques ou optiquement actives, ainsi que des sels d'addition avec les acides minéraux ou organiques pharmaceutiquement acceptables desdits produits de formule I.

Parmi ces médicaments, on retient notamment les produits tels que définis par la formule I ci-dessus, caractérisés en ce que dans la formule I, l'atome d'hydrogène en position 3 et le radical éthyle

en position 16 sont cis, ainsi que leurs sels d'addition avec les acides minéraux ou organiques et plus particulièrement le (3$\alpha$, 16$\alpha$) 14-imino -(15H) éburnaménine.

L'ensemble des produits tels que définis ci-dessus constituent des médicament très utiles en thérapeutique humaine, dans le traitement des vasculopathies cérébrales et de tous les syndromes provoqués par une altération de la circulation cérébrale; ils permettent de prévenir ou de diminuer les effets de l'artériosclérose cérébrale, des troubles circulatoires cérébraux, des hémorragies méningées ou cérébrales. Ils peuvent être utilisés en particulier dans le traitement des insuffisances cérébrales, des accidents cérébro-vasculaires et des traumatismes craniens.

La présente demande a enfin pour objet les compositions pharmaceutiques contenant, à titre de principe actif, l'un au moins des médicements tels que définis ci-dessus.

Ces compositions sont réalisées, de façon à pouvoir être administrées par la voie digestive ou parentérale. Elles peuvent être solides ou liquides et se présenter sous les formes pharmaceutiques couramment utilisées en médecine humaine comme, par exemple, les comprimés, simples ou dragéifiés, les gélules, les granulés, les suppositoires, les préparations injectables; elles sont préparées selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, le glycols, les divers agents mouillants, dispersants ou émulsifiants, les conservateurs.

La posologie usuelle, variable selon le produit utilisé, le sujet traité et l'affection en cause, peut être, par exemple, de 10 à 200 mg par jour chez l'adulte, par voie orale.

Les produits de formule II utilisés au départ du procédé de préparation des produits de formule I, décrit ci-dessus, ont été décrits par la demanderesse dans les brevets français 2.081.593 et 2 104 959.

Les sels des produits de formule II utilisés éventuellement au départ du procédé peuvent être préparés selon les méthodes usuelles à partir des produits de formule II.

Les exemples suivants illustrent l'invention sans toutefois la limiter.

Exemple 1: (3$\alpha$, 16$\alpha$) 14-imino-(15H) éburnaménine

On mélange 100 gr de chlorhydrate de (3$\alpha$, 164$\alpha$) 14,15 dihydro 15-hydroxylimino D- homo éburnaménin-14-one, 250 cm3 d'éthoxyéthanol, 32,1 g d'hydroxyde de sodium en pastilles, conserve sous agitation pendant une heure, chauffe ensuite jusqu'au reflux et maintient au reflux pendant 24 heures. On refroidit alors légèrement le milieu réactionnel et on verse lentementsur 1200 g de glace, conserve pendant une heure sous agitation, essore, lave à l'eau, sèche à 60°C en étuve les cristaux obtenus et obtient 69,2 g de produit attendu brut.

On purifie 20 g de ce produit par recristallisation dans 60 cm3 de méthanol, traite pendant 10 minutes par 0,4 g de charbon actif, filtre, rince deux fois par 5 cm3 de méthanol bouillant, refroidit jusqu'à une température de l'ordre de 0 à + 5°C et maintient une heure à cette température les cristaux obtenus, essore, lave par deux fois 10 cm3 de méthanol glacé, et obtient 12,2 g de produit attendu.

F = 161°C

$(\alpha)^{20}_{D}$ (c = 0,5% $CH\ Cl_3$) = −92° ± 1°5

analyse = ($C_{19}$ $H_{23}$ $N_3$)

| | | | |
|---|---|---|---|
| calculé: | C% 77,77; | H% 7,90; | N% 14,32 |
| trouvé | 78,1 | 7,9 | 14,0 |

Spectre ultra-violet dans l'éthanol

| | | | |
|---|---|---|---|
| max: | 242 nm | $E^1_1$ = 647 | $\varepsilon$ = 18 900 |
| max: | 274 nm | $E^1_1$ = 349 | $\varepsilon$ = 10 200 |
| max: | 292 nm | $E^1_1$ = 256 | $\varepsilon$ = 7 500 |
| max: | 302 nm | $E^1_1$ = 245 | $\varepsilon$ = 7 200 |

Le chlorhydrate de (3$\alpha$, 16$\alpha$) 14,15-dihydro 15-hydroxyimino D-homo éburnaménin-14-one utilisé au départ de l'exemple 1 a été préparé comme suit:

A une solution de 208,8 g de (3$\alpha$, 16$\alpha$) 14,15-dihydro 15-hydroxyimino D-homo éburnaménin-14-one dans 500 cm3 de toluène, on ajoute 2 litres d'acétone, puis on introduit sous agitation et sous argon en 10 à 15 minutes et à une température de 20—21°C.

58 cm3 d'acide chlorhydrique 22°Be (soit 42,8 g d'acide chlorhydrique pouc 100 cm3) ce qui revient à introduire 24,8 g d'acide pur.

Dés le début, le chlorhydrate cristallise. En fin d'additionon a une suspension jaune. On agite une heure à 20°C, essore, lave par empâtage 2 fois de suite avec à chaque fois 150 cm3 d'acétone. On sèche à 40° en étuve sous vide pendant 16 heures.

On obtient 219,8 g de chlorhydrate attendu, soit un rendement de 95% $(\alpha)^{20}_{D}$ (c = 1% dans la pyridine) = + 77° ± 2°

Exemple 2: (3α,16α) éburnaménin-14-(15 H)-one

On mélange 20 g de (3α, 16α) 14-imino-15 H)éburnaménine, obtenu à l'exemple 1, 100 cm3 de méthanol, 20 cm3 d'acide acétique, 10 cm3 d'eau déminéralisée, conserve la solution obtenue à 21—22°C pendant 48 heures, verse ensuite la solution dans 1000 cm3 d'eau froide. On ajoute 40 cm3 d'hydroxyde d'ammonium concentré, agite 30 minutes puis essore, lave à l'eau et sèche à 60°C en étuve, et obtient 19,85 g de produit attendu brut.

On purifie 20 g de ce produit brut de la manière suivante: on dissout les 20 g de produit dans 50 cm3 de chlorure de méthylène, filtre, rince au chlorure de méthylène, distille ensuite à pression normale et termine la distillation sous vide, reprend le résidu obtenu par 20 cm3 de méthanol à 20—222C, essore et lave avec 2 fois 10 cm3 de méthanol et obtient 19,1 g de produit attendu pur.

F = 174,5°C

$(\alpha)^{20}_{D}$ (c = 1% $CH\ Cl_3$) = —902 ± 2°

Exemple 3 = (3α, 16α) éburnaménin -14 (15 H)one

On mélange 800 cm3 d'éthoxy éthanol, 64,2 g d'hydroxyde de sodium en pastilles et 200 g de chlorhydrate de (3α, 16α) 14, 15 - dihydro - 15 - hydroxyimino - D - homo éburnaménin 14-one, agite la suspension obtenue pendant une heure, puis porte au reflux pendant 24 heurs. On distille alors l'éthoxy éthanol sous une pression de 10 mm/Hg et en maintenant à une température maximale de 70°C.

On reprend le résidue obtenu par 800 cm3 d'eau, ajoute ensuite lentement 200 cm3 d'acide chlorhydrique, obtient une solution que l'on chauffe à 90—95°C pendant 30 minutes, on verse sur 1 kg de glace et alcalinise par addition lente de 250 cm3 d'hydroxyde de sodium concentré; on extrait par une fois 500 cm3 et 4 fois 200 cm3 de chlorure de méthylène, réunit les extraits, sèche et amène a' sec, reprend le résidu par 100 cm3 de méthanol et amène à sec, reprend par 200 cm3 de méthanol et maintient au reflux pendant 15 minutes, ramène à 20°C, conserve 30 minutes à cette température, essore, lave par 3 fois 50 cm3 de méthanol, sèche à 60°C et obtient 138,4 g de produit attendu

F = 175°C

$(\alpha)^{20}_{D}$ (c = 1% $CH\ Cl_3$) = —922 ± 2°.

Le produit obtenu est de structure identique à celui obtenu à l'exemple 2.

On récupère, de plus 4,8 g du même produit attendu des eauxmères de cristallisation.

Exemple 4: (3α, 16α) éburnaménin-14-(15 H)-one

On introduit dans un autoclave 45 g de chlorhydrate de (3α, 16α) 14,15 - dihydro - 15 - hydroxylimino - D - homo éburnaménin - 14 - one, 2,4,6,7 cm3 d'hydroxyde de sodium normal, 1103 cm3 d'eau déminéralisée, maintient sous agitation à témpérature ambiante pendant une heure, chauffe de manière à ce que la température atteigne 130—135°C à l'intérieur de l'autoclave. La pression à l'intérieur de l'autoclave se stabilise à 2,5 kg ± 0,1. On conserve dans ces conditions pendant 24 heures, refroidit à 25°C. on extrait alors par 3 fois 150 cm3 de chlorure de méthylène, sèche et amène à sec, reprend le produit obtenu par 45 cm3 de méthanol, amène à sec, reprend par 45 cm3 de méthanol en agitant 30 minutes à 20—25°C, essore et rince par 3 fois 15 cm3 de méthanol.

On obtient 29,15 g de produit attendu

F = 175°C

$(\alpha)^{20}_{D}$ (c = 0,5% $CH\ Cl_3$) = —93° ± 2°

Le produit obtenu est de structure identique à celui obtenu aux exemples 2 et 3.

Exemple 5: (3α, 16α) éburnaménin 14-(15 H)-one

On mélange dans un autoclave 1,5 g de (3α, 16α)-14-imino-(15H)-éburnaménin, 10,48 cm3 d'une solution normale d'hydroxyde de sodium 26,2 cm3 d'eau déminéralisée. On chauffe à l'aide d'un bain d'huile à 150°C pendant 24 heures en maintenant sous agitation (la température à l'intérieur de l'autoclave est de 135°C et la pression de 2,5 kg). On refroidit ensuite l'appareil à 20°C, puis on ouvre l'autoclave. On recueille le produit par extraction au chlorure de méthylène, lave l'extrait à l'eau, sèche, filtre et amène à sec, reprend le résidue par 1,5 cm3 de méthanol, refroidit pendant une heure à 18—20°C, essore, rince, sèche et obtient 1,31 g de produit attendu.

F = 175°C

$(\alpha)^{20}_{D}$ (c = 1% chloroforme) = —91°5 ± 2°

Ce produit est de structure identique à celui obtenu aux exemples 2, 3 et 4.

Exemple 6: Formes pharmaceutiques

a) *comprimés* = on a préparé des comprimés répondant à la formule suivante:

— $(3\alpha, 16\alpha)$-14-imino-(15H)-éburnaménine 30 mg

— excipient q.s pour un comprimé (Détail de l'excipient: lactose, amidon de blé, amidon traité, amidon de riz, stéarate de magnésium, talc).

b) *gélules* = on a préparé des gélules des formule suivante:

— $(3\alpha, 16\alpha)$ -14-imino-(15H)-éburnaménine 30 mg

— excipient q.s pour une gélule terminée à 150 mg

(Détail de l'excipient: talc, stéarate de magnésium, aérosil).

Etude pharmacologique:

1) Détermination de la toxicité aiguë:

La toxicité aiguë est déterminée sur des lots de 5 ou 10 souris mâles d'un poids de 20—22 g, à jeun depuis cinq heures.

Le produit est administré par voie orale, en suspension dans une solution à 0,5% de méthylcellulose, au moyen d'une sonde oesophagienne.

La mortalité est relevée quotidiennement pendant une semaine. La dose léthale 50 (DL 50) à été déterminée et on a obtenu le résultat suivant:

| Produit de l'exemple | DL 50 mg/kg |
|---|---|
| 1 | > 1000 |

2) Test de l'anoxie hypobare:

On utilise des souris mâles d'un poids de 20—22 g, à jeun depuis cinq heures, réparties par groupe de 10 animaux.

On administre au animaux le produit à tester, par voie orale, en suspension dans une solution à 0,5% de méthylcellulose. Quinze minutes après administration du produit, on place les animaux dans un dessiccateur de 2 litres dans lequel on amène rapidement au moyen d'une pompe, la pression à 90 mm de Hg, et note leur temps de survie exprimé en secondes.

On note l'augmentation du temps de survie, exprimé en pourcentage des animaux traités par rapport aux animaux témoins, soumis aux mêmes conditions.

On a obtenu le résultat suivant:

| Produit de l'exemple | Dose | Augmentation du temps de survie % |
|---|---|---|
| 1 | 100 mg/kg | + 50 % |

3) Action sur le débit vertébral et le débit fémoral du chien.

Cette étude a été effectuée à thorax ouvert sur des chiens Beagle des deux sexes, pesant de 10 à 13 kg, anesthésiés au chloralose.

Le débit vertébral (exprimé en ml/mn) a été mesuré à l'aide d'un débitmètre électromagnétique STATHAM placé au départ de l'artère vertébrale droite.

Le débit fémoral a été mesuré dans les mêmes conditions au niveau de l'artère fémorale droite.

Par ailleurs, la pression artérielle et la fréquence cardiaque ont été enregistrées.

Les différents paramètres ont été mesurés avant et après injection du produit et on a calculé les variations maximales exprimées en pourcentages.

Dans le tableau suivant, n représente le nombre d'essais, pour chaque dose de produit.

| Produit | Doses (mg/kg) | Variations % | | | | | | | |
|---|---|---|---|---|---|---|---|---|---|
| | | Pression artérielle moyenne | n | Fréquence cardiaque | n | Débit vertébral | n | Débit fémoral | n |
| de l'exemple 1 | 0,1 | 4,7 ± 3,7 | 3 | −2,3 ± 2,3 | 3 | 26 ± 3,8 | 3 | 27,7 ± 8,9 | 3 |
| | 0,3 | 2,3 ± 3,8 | 6 | −2 ± 4,4 | 6 | 28 ± 15,5 | 6 | 17,3 ± 18,2 | 6 |
| Vincamine | 0,1 | −6,5 ± 6,5 | 2 | −10 ± 10 | 2 | 5 ± 5 | 2 | 17 | 1 |
| | 0,3 | −0,6 ± 1,4 | 5 | −15,2 ± 4,3 | 5 | 1,0 ± 11,5 | 5 | −2,8 ± 2,8 | 4 |

Conclusion

Le produit de l'exemple 1 entraîne dès la dose 0,1 mg/kg I.V., une augmentation du débit vertébral, sans qu'il y ait modification sensible de la pression artérielle et de la fréquence cardiaque. Par ailleurs, il entraîne une augmentation du débit fémoral.

La vincamine aux mêmes doses, modifie le débit vertébral et le débit fémoral de façon irrégulière (comme le montrent les valeurs d'écart-type à la moyenne), et provoque une légère diminution de la fréquence cardiaque.

**Revendications:**

1. Les produits de formule

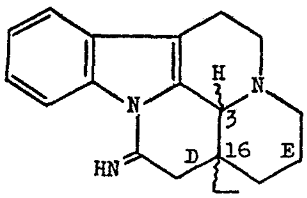

(I).

lesdits produits de formule I étant sous leurs formes racémiques ou optiquement actives, ainsi que les sels d'addition avec les acides minéraux ou organiques desdits produits de formule I.

2. Les produits tels que définis par la formule I de la revendication 1, caractérisés en ce que dans la formule I, l'atome d'hydrogène en position 3 et le radical éthyle en position 16 sont cis, ainsi que leurs sels d'addition avec les acides minéraux ou organiques.

3. Le ($3\alpha$, $16\alpha$) 14-imino-(15H) éburnaménine.

4. Procédé de préparation des produits tels que définis par la formule I de la revendication 1, sous leurs formes racémiques ou optiquement actives, ainsi que des sels d'addition avec les acides minéraux ou organiques desdits produits de formule I, caractérisé en ce que l'on traite un produit de formule

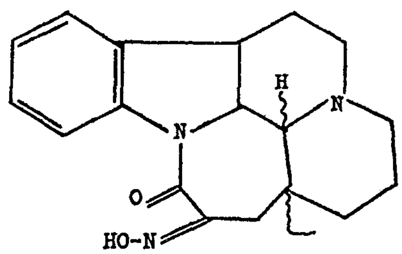

(II)

ou un de ses sels, par un agent basique, pour obtenir un produit de formule I correspondant que, si désiré, on traite par un acide minéral ou organique pour en former le sel.

5. Procédé, selon la revendication 4, de préparation du ($3\alpha$, $16\alpha$) 14-imino-(15H) éburnaménine et de ses sels, caractérisé en ce que l'on utilise comme produit de formule II la ($3\alpha$, $16\alpha$) 14,15-dihydro 15-hydroxyimino-D-homo éburnaménin-14-one ou l'un de ses sels.

6. Procédé selon la revendication 4 ou 5, caractérisé en ce que l'agent basique est un hydroxyde de métal alcalin.

7. Procédé selon la revendication 4 ou 5, caractérisé en ce que la réaction du produit de formule II et de l'agent basique est réalisée à une température allant de 100 à 150°C.

8. Application des produits de formule I, telle que définie à la revendication 1, et de leurs sels d'addition avec les acides minéraux ou organiques, à la préparation des produits du groupe de l'éburnamonine, de formule:

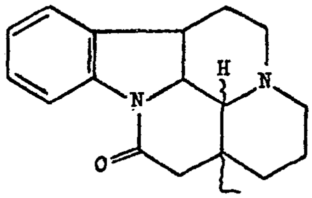

(III)

sous leurs formes racémiques ou optiquement actives, caractérisée en ce que l'on soumet un produit de formule I, ou un de ses sels, à l'action d'un agent d'hydrolyse acide ou basique, pour obtenir un produit de formule III sous forme salifiée ou non, et que, dans le cas ou l'on obtient un sel de produit de formule III, l'on traite, le cas échéant, par une base pour obtenir le produit de formule III correspondant.

9. Application selon la revendication 8, caractérisée en ce que le produit de formule I de départ est préparé selon le procédé de la revendication 4.

10. Application selon la revendication 8 ou 9 à la préparation de la ($3\alpha$, $16\alpha$) éburnaménin-14

(15H)-one, caractérisée en ce que l'on utilise comme produit de formule I la ($3\alpha$, $16\alpha$) 14-imino (15H)-éburnaménine.

11. Application selon la revendication 8, 9 ou 10, qui consiste à préparer un produit de formule I, et qui est caractérisée en ce que, sans être isolé, le produit de formule I est transformé en produit de formule III.

12. Application selon la revendication 9 ou 10, caractérisée en ce que le produit de formule I est préparé en milieu aqueux selon le procédé de la revendication 4, et que l'hydrolyse dudit produit de formule I est réalisée au fur et à mesure de sa préparation.

13. Compositions pharmaceutiques, caractérisées en ce qu'elles renferment, à titre de principe actif, l'un au moins des produits de formule I, telle que définie à la revendication 1, sous leurs formes racémiques ou optiquement actives, ou l'un au moins de leurs sels d'addition avec les acides minéraux ou organiques pharmaceutiquement acceptables.

14. Compositions pharmaceutiques, caractérisées en ce qu'elles referment, à titre de principe actif, l'un au moins des produits de formule I, telle que définie à la revendication 2 ou 3, sous leurs formes racémiques ou optiquement actives ou l'un au moins de leurs sels d'addition avec les acides minéraux ou organiques pharmaceutiquement acceptables.

**Claims**

1. The products of formula I:

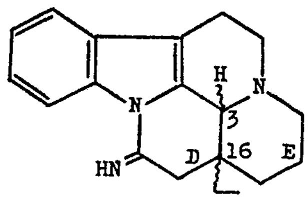

(I)

the said products of formula I being in their racemic or optically-active forms, as well as the addition salts with mineral or organic acids of the said products of formula I.

2. The products as defined by the formula I of Claim 1, characterised in that in the formula I the hydrogen atom at position 3 and the ethyl radical at position 16 are *cis*, as well as their addition salts with mineral or organic acids.

3. ($3\alpha$,$16\alpha$)14-imino-(15H)-eburnamenine,

4. Process for preparing the products as defined by the formula I of Claim 1 in their racemic or optically-active forms, as well as addition salts with mineral or organic acids of the said products of formula I, characterised in that a product of formula II:

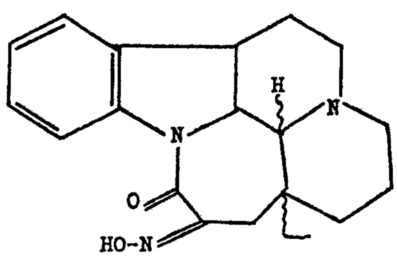

(II)

or one of its salts is treated with a basic agent to obtain a corresponding product of formula I which, if desired, is treated with a mineral or organic acid to form the salt thereof.

5. Process according to Claim 4 for preparing ($3\alpha$,$16\alpha$) 14-imino-(15H) eburnamenine and its salts, characterised in that, as product of formula II, ($3\alpha$,$16\alpha$) 14,15-dihydro 15-hydroxyimino-D-homo eburnamenin-14-one or one of its salts is used.

6. Process according to Claim 4 or 5, characterised in that the basic agent is an alkali-metal hydroxide.

7. Process according to claim 4 or 5, characterised in that the reaction of the product of formula II and of the basic agent is carried out at a temperature ranging from 100 to 150°C.

8. Use of the products of formula I as defined in Claim 1 and of their addition salts with mineral or organic acids in the preparation of the products of the eburnamonine group, of formula III:

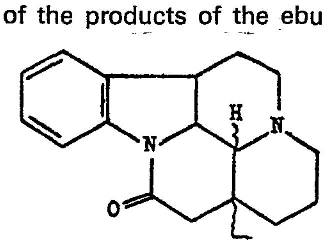

(III)

in their racemic or optically-active forms, characterised in that a product of formula I or one of its salts is subjected to the action of an acidic or basic hydrolysis agent to obtain a product of formula III in salified or unsalified form and in that, in the case in which a salt of the product of formula III is obtained, it is treated, if appropriate, with a base to obtain the corresponding product of formula III.

9. Use according to Claim 8, characterised in that the starting product of formula I is prepared according to the process of Claim 4.

10. Use according to Claim 8 or 9 in the preparation of (3$\alpha$, 16$\alpha$) eburnamenin-14 (15H)-one, characterised in that, as product of formula I (3$\alpha$16$\alpha$) 14 - imino - (15H) - eburnamenine is used.

11. Use according to Claim 8, 9 or 10 which consists in preparing a product of formula I and which is characterised in that, without being isolated, the product of formula I is converted into a product of formula III.

12. Use according to Claim 9 or 10, characterised in that the product of formula I is prepared in aqueous medium according to the process of Claim 4 and in that the hydrolysis of the said product of formula I is carried out in the course of its preparation.

13. Pharmaceutical compositions, characterised in that they contain, as active principle, one at least of the products of formula I as defined in Claim 1, in their racemic or optically-active forms, or one at least of their addition salts with the pharmaceutically-acceptable mineral or organic acids.

14. Pharmaceutical compositions, characterised in that they contain, as active principle, one at least of the products of formula I as defined in Claim 2 or 3, in their racemic or optically-active forms, or one at least of their addition salts with the pharmaceutically-acceptable mineral or organic acids.

**Patentansprüche**

1. Produkte der Formel (I)

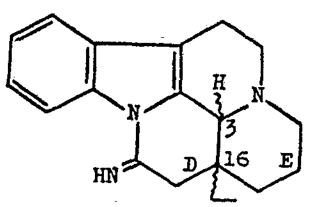

(I)

wobei diese Produkte der Formel (I) in ihren racemischen oder optisch aktiven Formen vorliegen können, sowie die Additionssalze mit Mineral- oder organischen Säuren der Produkte der Formel (I).

2. Produkte der Formel (I) gemäß Anspruch 1, dadurch gekennzeichnet, daß in der Formel (I) das Wasserstoffatom in 3-Stellung und der Äthylrest in 16-Stellung cis-ständig sind, sowie deren Additionssalze mit Mineral- oder organischen Säuren.

3. (3$\alpha$,16$\alpha$)-14-Imino-(15H)-eburnamenin.

4. Verfahren zur Herstellung der Produkte der Formel (I) gemäß Anspruch 1, in ihren racemischen oder optisch aktiven Formen sowie der Additionssalze mit Mineral- oder organischen Säuren der Produkte der Formel (I), dadurch gekennzeichnet, daß man ein Produkt der Formel (II)

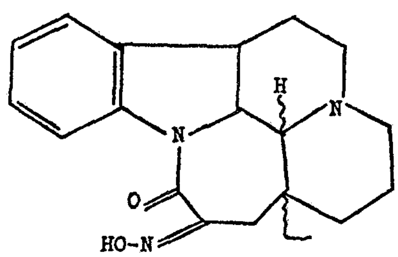

(II)

oder eines seiner Salze mit einem basischen Mittel behandelt, um ein entsprechendes Produkt der Formel (I) zu erhalten, das man gewünschtenfalls mit einer Mineral- oder organischen Säure behandelt, um hieraus das Salz zu bilden.

5. Verfahren gemäß Anspruch 4 zur Herstellung von (3$\alpha$,16$\alpha$) - 14 - Imino - (15H) - eburnamenin und dessen Salzen, dadurch gekennzeichnet, daß man als Produkt der Formel (II) das (3$\alpha$,16$\alpha$) - 14,15 - Dihydro - 15 - hydroxyimino - D - homo - eburnamenin - 14 - on oder eines seiner Salze verwendet.

6. Verfahren gemäß Anspruch 4 oder 5, dadurch gekennzeichnet, daß das basische Mittel ein Alkalmetallhydroxyd ist.

7. Verfahren gemäß Anspruch 4 oder 5, dadurch gekennzeichnet, daß die Umsetzung des Produkts der Formel (II) und des basischen Mittels bei einer Temperatur von 100—150°C durchgeführt wird.

8. Verwendung der Produkte der Formel (I) gemäß Anspruch 1, und von deren Additionssalzen

mit Mineral- oder organischen Säuren zur Herstellung von Produkten der Gruppe des Eburnamonins der Formel (III)

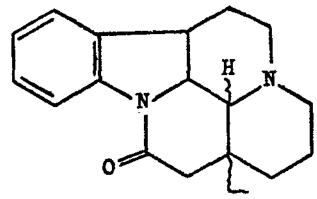

(III)

in deren racemischen oder optisch aktiven Formen, dadurch gekennzeichnet, daß man ein Produkt der Formel (I) oder eines seiner Salze der Einwirkung eines sauren oder basischen Hydrolysemittels unterzieht, um ein Produkt der Formel (III) in der in ein Salz überführten oder nicht überführten Form zu erhalten und daß man, wenn man ein Salz des Produkts der Formel (III) erhält, man dieses gegebenenfalls mit einer Base behandelt, um das entsprechende Produkt der Formel (III) zu erhalten.

9. Verwendung gemäß Anspruch 8, dadurch gekennzeichnet, daß das Ausgangsprodukt der Formel (I) gemäß dem Verfahren von Anspruch 4 hergestellt wird.

10. Verwendung gemäß Anspruch 8 oder 9 zur Herstellung von (3$\alpha$,16$\alpha$) - Eburnamenin - 14 - (15H) - on, dadurch gekennzeichnet, daß man als Produkt der Formel (I) das (3$\alpha$,16$\alpha$) - 14 - Imino - (15H) - eburnamenin einsetzt.

11. Verwendung gemäß Anspruch 8, 9 oder 10, die in der Herstellung eines Produkts der Formel (I) besteht und die dadurch gekennzeichnet ist, daß man ohne Isolierung das Produkt der Formel (I) in ein Produkt der Formel (III) überführt.

12. Verwendung gemäß Anspruch 9 oder 10, dadurch gekennzeichnet, daß das Produkt der Formel (I) in wäßrigem Milieu gemäß dem Verfahren von Anspruch 4 hergestellt wird, und daß die Hydrolyse des Produkts der Formel (I) nach Maßgabe seiner Herstellung bewirkt wird.

13. Pharmazeutische Zusammensetzungen, dadurch gekennzeichnet, daß sie als Wirkstoff zumindest eines der Produkte der Formel (I) gemäß Anspruch 1 in ihren racemischen oder optisch aktiven Formen oder zumindest eines ihrer pharmazeutisch verträglichen Additionssalze mit Mineral- oder organischen Säuren enthalten.

14. Pharmazeutische Zusammensetzungen, dadurch gekennzeichnet, daß sie als Wirkstoff zumindest eines der Produkte der Formel (I) gemäß Anspruch 2 oder 3 ihren racemischen oder optischen aktiven Formen oder zumindest eines der pharmazeutisch verträglichen Additionssalze mit Mineral- oder organischen Säuren enthalten.